(19) Europäisches Patentamt
European Patent Office
Office européen des brevets

(11) Veröffentlichungsnummer: **0 336 132**
**A1**

(12) # EUROPÄISCHE PATENTANMELDUNG

(21) Anmeldenummer: 89104076.8

(51) Int. Cl.⁴: **C07D 211/90 , A61K 31/445**

(22) Anmeldetag: 08.03.89

(30) Priorität: 11.03.88 CH 921/88
11.03.88 CH 931/88

(43) Veröffentlichungstag der Anmeldung:
11.10.89 Patentblatt 89/41

(84) Benannte Vertragsstaaten:
ES GR

(71) Anmelder: Byk Gulden Lomberg Chemische Fabrik GmbH
Byk-Gulden-Strasse 2
D-7750 Konstanz(DE)

(72) Erfinder: Flockerzi, Dieter, Dr.
Ackerweg 26
D-7753 Allensbach(DE)
Erfinder: Ulrich, Wolf-Rüdiger, Dr.
Hebelstrasse 2
D-7750 Konstanz(DE)
Erfinder: Sanders, Karl, Dr.
Felchengang 23
D-7750 Konstanz(DE)
Erfinder: Beller, Klaus-Dieter
Franz-Moser-Strasse 5
D-7750 Konstanz 16(DE)
Erfinder: Boer, Rainer, Dr.
Löhrystrasse 4
D-7750 Konstanz(DE)
Erfinder: Eltze, Manfrid, Dr.
Schützenstrasse 20
D-7750 Konstanz(DE)
Erfinder: Klemm, Kurt, Prof. Dr.
Im Weinberg 2
D-7753 Allensbach(DE)

(54) **Neue 1-Phenylpropylverbindungen.**

(57) Verbindungen der Formel I

EP 0 336 132 A1

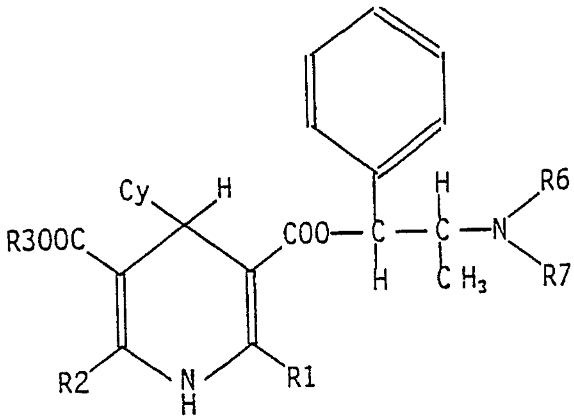

(I)

worin die Substituenten und Symbole die in der Beschreibung genannten Bedeutungen haben, sind neue Verbindungen mit überraschenden pharmakologischen Eigenschaften.

# Neue 1-Phenylpropylverbindungen

## Anwendungsgebiet der Erfindung

Die Erfindung betrifft neue 1-Phenylpropylverbindungen. Verfahren zu ihrer Herstellung, ihre Verwendung und sie enthaltende Arzneimittel. Die erfindungsgemäßen Verbindungen werden in der pharmazeutischen Industrie als Zwischenprodukte und zur Herstellung von Arzneimitteln eingesetzt.

## Bekannter technischer Hintergrund

Es ist bekannt, daß bestimmte, auf verschiedene Weise substituierte 1,4-Dihydropyridinderivate pharmakologisch nützliche Eigenschaften aufweisen. In den Europäischen Patentanmeldungen 179 386, 222 702, 88 903, 60 897, 244 595 und 128 010 werden solche 1,4-Dihydropyridin-3,5-dicarbonsäuredialkylester offenbart, bei denen einer der Alkylesterreste durch eine als basischer Rest fungierende substituierte Aminogruppe substituiert ist. Überraschenderweise wurde nun gefunden, daß die unten näher beschriebenen neuen 1,4-Dihydropyridine mit basischem Rest besonders interessante pharmakologische Eigenschaften aufweisen, durch die sie sich von den Verbindungen des Standes der Technik in vorteilhafter Weise unterscheiden.

## Beschreibung der Erfindung

Gegenstand der Erfindung sind neue 1-Phenylpropylverbindungen der Formel I

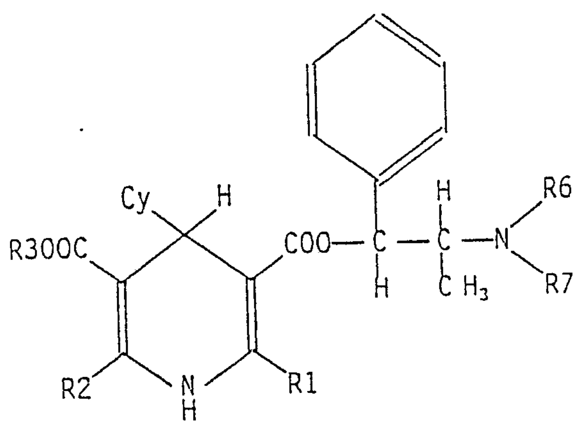

(I)

worin Cy einen Cyclus der Formel

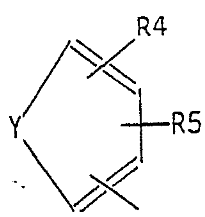

darstellt, in dem Y Sauerstoff (O), Schwefel (S), Vinylen (-CH = CH-), Azomethin (-CH = N-) oder eine Gruppe der Formel

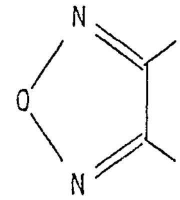

oder

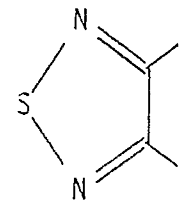

bedeutet.

R1 1-6C-Alkyl oder 3-7C-Alkoxyalkyl bedeutet,

R2 1-6C-Alkyl oder 3-7C-Alkoxyalkyl bedeutet,

R3 Wasserstoff, 1-6C-Alkyl oder 3-7C-Alkoxyalkyl bedeutet,

R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino, Mono- oder Di-1-4C-alkylamino oder gemeinsam Methylendioxy bedeuten,

R6 Wasserstoff, 1-6C-Alkyl, 1-4C-Alkoxycarbonyl, 2-5C-Acyl, Arylcarbonyl, Furoyl oder eine in der Peptidchemie gebräuchliche N-Schutzgruppe bedeutet und

R7 Wasserstoff, 1-6C-Alkyl, Aryl-1-4C-alkyl, Aryl-2-4C-alkenyl oder Aryl-2-4C-alkinyl bedeutet, wobei

Aryl für Phenyl, substituiertes Phenyl mit einem oder zwei Substituenten aus der Gruppe Halogen, 1-4C-Alkyl, 1-4C-Alkoxy, Hydroxy oder Nitro oder substituiertes Phenyl mit einem, zwei oder drei Substituenten aus der Gruppe 1-4C-Alkoxy steht,

und die Salze dieser Verbindungen.

1-6C-Alkyl ist geradkettig oder verzweigt und bedeutet beispielsweise einen Hexyl-, Neopentyl-, Isopentyl-, Butyl-, i-Butyl-, sec.-Butyl-, t-Butyl-, Propyl-, Isopropyl- oder insbesondere Ethyl- oder Methylrest.

3-7C-Alkoxyalkyl steht beispielsweise für einen Ethoxyethyl-, Propoxyethyl-, Isopropoxyethyl-, Butoxyethyl-, Methoxypropyl-, 2-Methoxy-1-methylethyl-, 2-Ethoxy-1-methylethyl- oder insbesondere Methoxyethylrest.

Halogen im Sinne der Erfindung bedeutet Brom, Fluor und insbesondere Chlor.

1-4C-Alkyl ist geradkettig oder verzweigt und bedeutet beispielsweise einen Butyl-, i-Butyl-, sec.-Butyl-, t-Butyl-, Propyl-, Isopropyl-, Ethyl- oder insbesondere Methylrest.

1-4C-Alkoxy enthält neben dem Sauerstoffatom einen der vorstehend genannten 1-4C-Alkylreste. Bevorzugt sind der Methoxy- und der Ethoxyrest.

Ganz oder teilweise durch Fluor substituiertes 1-4C-Alkoxy ist beispielsweise 1,1,2,2-Tetrafluorethoxy, Trifluormethoxy, 2,2,2-Trifluorethoxy oder insbesondere Difluormethoxy.

1-4C-Alkoxycarbonyl enthält neben der Carbonylgruppe einen der vorstehend genannten 1-4C-Alkoxyreste. Bevorzugt sind der Methoxycarbonyl- und der Ethoxycarbonylrest.

2-5C-Acyl enthält neben der Carbonylgruppe einen der vorstehend genannten 1-4C-Alkylreste. Bevorzugt ist der Acetylrest.

Mono- oder Di-1-4C-alkylamino enthält neben dem Stickstoffatom einen oder zwei der vorstehend genannten 1-4C-Alkylreste. Bevorzugt ist Di-1-4C-alkylamino, und hier insbesondere Dimethyl-, Diethyl- oder Diisopropylamino.

Arylcarbonyl steht für eine Carbonylgruppe, die durch Aryl substituiert ist. Als bevorzugte Arylcarbonylgruppen seien beispielsweise die 4-Chlorphenylcarbonyl, 2-Hydroxyphenylcarbonyl- (Salicyloyl-), die 4-Hydroxyphenylcarbonyl- und insbesondere die 4-Fluorphenylcarbonyl- und die Benzoylgruppe genannt.

Als in der Peptidchemie gebräuchliche N-Schutzgruppen seien beispielsweise die Triphenylmethylgruppe, die Nitrophenylsulfenylgruppe, die Benzyloxycarbonylgruppe oder insbesondere die tert.-Butoxycarbonylgruppe genannt.

Aryl-1-4C-alkyl steht für 1-4C-Alkyl, das durch Aryl substituiert ist. Als beispielhafte bevorzugte Aryl-1-4C-alkylreste seien genannt die Reste: 4-Methylbenzyl, 4-Methoxybenzyl, 4-Chlorbenzyl, 1-Phenethyl, 2-Phenylethyl, 3-Chlorbenzyl, 2,5-Dimethylbenzyl, 4-Fluorbenzyl, 3-Methylbenzyl und insbesondere Benzyl.

Aryl-2-4C-alkenyl und Aryl-2-4C-alkinyl steht für Alkenyl- und Alkinylreste mit 2 bis 4 Kohlenstoffatomen, die durch Aryl substituiert sind. Beispielsweise seien der 3-Phenyl-2-propenylrest und der 3-Phenyl-2-propinylrest genannt.

Als Salze kommen alle Salze mit Säuren in Betracht. Besonders erwähnt seien die pharmakologisch verträglichen Salze der in der pharmazeutischen Industrie üblicherweise verwendeten anorganischen und organischen Säuren. Pharmakologisch unverträgliche Salze, die beispielsweise bei der Herstellung der

erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Metembonat, Stearat, Tosilat, 2-Hydroxy-3-naphthoat, 3-Hydroxy-2-naphthoat oder Mesilat, aber auch Salze mit Bumetanid, Furosemid, Azosemid, Galosemid, Besunid, Piretanid, Etacrynsäure, Tienilinsäure oder 4-Chlor-sulfamoyl-benzoesäure.

Erwähnenswerte erfindungsgemäße Verbindungen sind solche der Formel I, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 2,3-Methylendioxyphenyl oder 2,1,3-Benzoxdiazol-4-yl bedeutet,
R1 Methyl, Ethyl oder Methoxyethyl bedeutet,
R2 Methyl, Ethyl oder Methoxyethyl bedeutet,
R3 Methyl, Ethyl oder Methoxyethyl bedeutet,
R6 Wasserstoff, Methyl, Ethyl, tert.-Butoxycarbonyl oder Acetyl bedeutet und
R7 Wasserstoff, Methyl, Benzyl, Chlorbenzyl, Brombenzyl, Methoxybenzyl, Dimethoxybenzyl, Trimethoxybenzyl, Hydroxybenzyl, Methlbenzyl, Dimethylbenzyl,Nitrobenzyl, methoxy-nitrobenzyl oder 3-Phenyl-2-propenyl bedeutet,
und die Salze dieser Verbindungen.

Besonders erwähnenswerte erfindungsgemäße Verbindungen sind solche der Formel I, worin
Cy 3-Nitrophenyl, 2-Chlorphenyl, 2,3-Dichlorphenyl, 2-Trifluormethylphenyl oder 2,1,3-Benzoxdiazol-4-yl bedeutet,
R1 Methyl oder Ethyl bedeutet,
R2 Methyl oder Ethyl bedeutet,
R3 Methyl, Ethyl oder Methyoxyethyl bedeutet,
R6 Wasserstoff, Methyl, Ethyl, tert.-Butoxycarbonyl order Acetyl bedeutet und
R7 Wasserstoff, Methyl, Benzyl, 3,4,5-Trimethoxybenzyl,2-ethoxy-5-nitrobenzyl der 3-Phenyl-2-propenyl bedeutet,
und ihre Salze.

Hervorzuhebende erfindungsgemäße Verbindungen sind solche der Formel I, worin
Cy 3-Nitrophenyl bedeutet,
R1 methyl bedeutet,
R2 Methyl bedeutet,
R3 Methyl oder Ethyl bedeutet,
R6 Wasserstoff, Methyl, Ethyl, tert.-Butoxycarbonyl oder Acetyl bedeutet und
R7 Wasserstoff, Methyl, Benzyl, Trimethoxybenzyl, Methoxy-nitrobenzyl oder 3-Phenyl-2-propenyl bedeutet,
und die Salze diesser Verbindungen.

Besonders hervorzuhebende erfindungsgemäße Verbindungen sind solche der Formel I, worin
Cy 3-Nitrophenyl bedeutet,
R1 Methyl bedeutet,
R2 methyl bedeutet,
R3 Methyl oder Ethyl bedeutet,
R6 Wasserstoff, Methyl oder Ethyl bedeutet und
R7 Methyl, Benzyl, 3,4,5-Trimethoxybenzyl, 2-Methoxy-5-nitrobenzyl oder 3-Phenyl-2-propenyl bedeutet,
und ihre Salze

Bevorzugte erfindungsgemäße Verbindungen sind solche der Formel I, worin
Cy 3-Nitrophenyl bedeutet,
R1 Methyl bedeutet,
R2 Methyl bedeutet,
R3 Methyl oder Ethyl bedeutet,
R6 Wasserstoff oder Methyl bedeutet und
R7 Methyl oder Benzyl bedeutet,
und ihre Salze.

Besonders bevorzugte erfindungsgemäße Verbindungen sind solche der Formel I, worin

Cy 3-Nitrophenyl,
R1 Methyl,
R2 Methyl,
R3 Methyl,
R6 Wasserstoff und
R7 Methyl bedeutet,
und ihre Salze.

Die Verbindungen der Formel I besitzen an der 4-Position im 1,4-Dihydropyridin ein Chiralitätszentrum und in der Seitenkette zwei weitere Chiralitätszentren. Die Erfindung umfaßt daher sowohl alle acht denkbaren Konfigurationsisomeren, als auch deren Gemische. Besonders bevorzugt sind in diesem Zusammenhang die Konfigurationsisomeren, die in der 4-Position im Dihydropyridin die gleiche Konfiguration aufweisen wie das als Ausgangsverbindung einsetzbare (-)-1-Ethoxymethyl-1,4-dihydro-5-methoxycarbonyl-2,6-dimethyl-4-(3-nitrophenyl)-pyridin-3-carbonsäure-cinchonidinsalz, das das linear polarisierte Licht der Wellenlänge 589 nm mit $[\alpha]_D^{22}$ = - 63,4° (c = 1, Chloroform) dreht.

Die besonders bevorzugten Konfigurationsisomeren können auch durch die folgende Formel Ia beschrieben werden

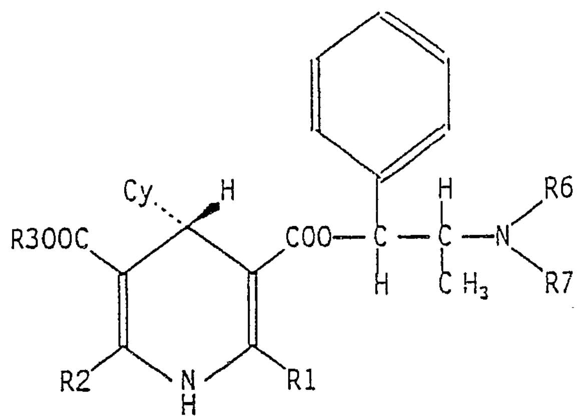

(Ia)

worin Cy, R1, R2, R3, R4, R5, R6 und R7 die oben angegebenen Bedeutungen haben und wobei die Anordnung der Substituenten Cy und H in 4-Position des 1,4-Dihydropyridinringes aufgrund der Veröffentlichung von K. Tamazawa et al., J. Med. Chem. 29, 2504 (1986) vorgenommen wurde.

Als hervorzuhebende erfindungsgemäße Verbindungen seien beispielsweise genannt:

1,4-Dihydro-2,6-dimethyl-4-(3-nitrophenyl)-pyridin-3,5-dicarbonsäure-3-methyl-5-[(1R),(2S)-2-dimethylamino-1-phenylpropyl-1]-ester

1,4-Dihydro-2,6-dimethyl-4-(3-nitrophenyl)-pyridin-3,5-dicarbonsäure-3-ethyl-5-[(1R),(2S)-2-dimethylamino-1-phenylpropyl-1]-ester

1,4-Dihydro-2,6-dimethyl-4-(3-nitrophenyl)-pyridin-3,5-dicarbonsäure-3-(2-methoxyethyl)-5-[(1R),(2S)-2-dimethylamino-1-phenylpropyl-1]-ester

1,4-Dihydro-2,6-dimethyl-4-(3-nitrophenyl)-pyridin-3,5-dicarbonsäure-3-(propyl-2)-5-[(1R),(2S)-2-dimethylamino-1-phenylpropyl-1]-ester

1,4-Dihydro-2,6-dimethyl-4-(3-nitrophenyl)-pyridin-3,5-dicarbonsäure-3-methyl-5-[(1S),(2R)-2-dimethylamino-1-phenylpropyl-1]-ester

1,4-Dihydro-2,6-dimethyl-4-(3-nitrophenyl)-pyridin-3,5-dicarbonsäure-3-ethyl-5-[(1S),(2R)-2-dimethylamino-1-phenylpropyl-1]-ester

1,4-Dihydro-2,6-dimethyl-4-(3-nitrophenyl)-pyridin-3,5-dicarbonsäure-3-(2-methoxyethyl)-5-[(1S),(2R)-2-dimethylamino-1-phenylpropyl-1]-ester

1,4-Dihydro-2,6-dimethyl-4-(3-nitrophenyl)-pyridin-3,5-dicarbonsäure-3-(propyl-2)-5-[(1S),(2R)-2-dimethylamino-1-phenylpropyl-1]-ester

4-(2,3-Dichlorphenyl)-1,4-dihydro-2,6-dimethyl-pyridin-3,5-dicarbonsäure-3-methyl-5-[(1R),(2S)-2-dimethylamino-1-phenylpropyl-1]-ester

4-(2,3-Dichlorphenyl)-1,4-dihydro-2,6-dimethyl-pyridin-3,5-dicarbonsäure-3-ethyl-5-[(1R),(2S)-2-dimethylamino-1-phenylpropyl-1]-ester

4-(2,3-Dichlorphenyl)-1,4-dihydro-2,6-dimethyl-pyridin-3,5-dicarbonsäure-3-(2-methoxyethyl)-5-[(1R),(2S)-2-dimethylamino-1-phenylpropyl-1]-ester

4-(2,3-Dichlorphenyl)-1,4-dihydro-2,6-dimethyl-pyridin-3,5-dicarbonsäure-3-(propyl-2)-5-[(1R),(2S)-2-dimethylamino-1-phenylpropyl-1]-ester
4-(2,3-Dichlorphenyl)-1,4-dihydro-2,6-dimethyl-pyridin-3,5-dicarbonsäure-3-methyl-5-[(1S),(2R)-2-dimethylamino-1-phenylpropyl-1]-ester
4-(2,3-Dichlorphenyl)-1,4-dihydro-2,6-dimethyl-pyridin-3,5-dicarbonsäure-3-ethyl-5-[(1S),(2R)-2-dimethylamino-1-phenylpropyl-1]-ester
4-(2,3-Dichlorphenyl)-1,4-dihydro-2,6-dimethyl-pyridin-3,5-dicarbonsäure-3-(2-methoxyethyl)-5-[(1S),(2R)-2-dimethylamino-1-phenylpropyl-1]-ester
4-(2,3-Dichlorphenyl)-1,4-dihydro-2,6-dimethyl-pyridin-3,5-dicarbonsäure-3-(propyl-2)-5-[(1S),(2R)-2-dimethylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1R),(2S)-2-dimethylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1S),(2R)-2-dimethylamino-1-phenylpropyl-1]-ester
1,4-Dihydro-2,6-dimethyl-4-(3-nitrophenyl)-pyridin-3,5-dicarbonsäure-3-methyl-5-[(1R),(2S)-2-methylamino-1-phenylpropyl-1]-ester
1,4-Dihydro-2,6-dimethyl-4-(3-nitrophenyl)-pyridin-3,5-dicarbonsäure-3-ethyl-5-[(1R),(2S)-2-methylamino-1-phenylpropyl-1]-ester
1,4-Dihydro-2,6-dimethyl-4-(3-nitrophenyl)-pyridin-3,5-dicarbonsäure-3-(2-methoxyethyl)-5-[(1R),(2S)-2-methylamino-1-phenylpropyl-1]-ester
1,4-Dihydro-2,6-dimethyl-4-(3-nitrophenyl)-pyridin-3,5-dicarbonsäure-3-(propyl-2)-5-[(1R),(2S)-2-methylamino-1-phenylpropyl-1]-ester
1,4-Dihydro-2,6-dimethyl-4-(3-nitrophenyl)-pyridin-3,5-dicarbonsäure-3-methyl-5-[(1S),(2R)-2-methylamino-1-phenylpropyl-1]-ester
1,4-Dihydro-2,6-dimethyl-4-(3-nitrophenyl)-pyridin-3,5-dicarbonsäure-3-ethyl-5-[(1S),(2R)-2-methylamino-1-phenylpropyl-1]-ester
1,4-Dihydro-2,6-dimethyl-4-(3-nitrophenyl)-pyridin-3,5-dicarbonsäure-3-(2-methoxyethyl)-5-[(1S),(2R)-2-methylamino-1-phenylpropyl-1]-ester
1,4-Dihydro-2,6-dimethyl-4-(3-nitrophenyl)-pyridin-3,5-dicarbonsäure-3-(propyl-2)-5-[(1S),(2R)-2-methylamino-1-phenylpropyl-1]-ester
4-(2,3-Dichlorphenyl)-1,4-dihydro-2,6-dimethyl-pyridin-3,5-dicarbonsäure-3-methyl-5-[(1R),(2S)-2-methylamino-1-phenylpropyl-1]-ester
4-(2,3-Dichlorphenyl)-1,4-dihydro-2,6-dimethyl-pyridin-3,5-dicarbonsäure-3-ethyl-5-[(1R),(2S)-2-methylamino-1-phenylpropyl-1]-ester
4-(2,3-Dichlorphenyl)-1,4-dihydro-2,6-dimethyl-pyridin-3,5-dicarbonsäure-3-(2-methoxyethyl)-5-[(1R),(2S)-2-methylamino-1-phenylpropyl-1]-ester
4-(2,3-Dichlorphenyl)-1,4-dihydro-2,6-dimethyl-pyridin-3,5-dicarbonsäure-3-(propyl-2)-5-[(1R),(2S)-2-methylamino-1-phenylpropyl-1]-ester
4-(2,3-Dichlorphenyl)-1,4-dihydro-2,6-dimethyl-pyridin-3,5-dicarbonsäure-3-methyl-5-[(1S),(2R)-2-methylamino-1-phenylpropyl-1]-ester
4-(2,3-Dichlorphenyl)-1,4-dihydro-2,6-dimethyl-pyridin-3,5-dicarbonsäure-3-ethyl-5-[(1S),(2R)-2-methylamino-1-phenylpropyl-1]-ester
4-(2,3-Dichlorphenyl)-1,4-dihydro-2,6-dimethyl-pyridin-3,5-dicarbonsäure-3-(2-methoxyethyl)-5-[(1S),(2R)-2-methylamino-1-phenylpropyl-1]-ester
4-(2,3-Dichlorphenyl)-1,4-dihydro-2,6-dimethyl-pyridin-3,5-dicarbonsäure-3-(propyl-2)-5-[(1S),(2R)-2-methylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1R),(2S)-2-methylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1S),(2R)-2-methylamino-1-phenylpropyl-1]-ester
1,4-Dihydro-2,6-dimethyl-4-(3-nitrophenyl)-pyridin-3,5-dicarbonsäure-3-methyl-5-[(1R),(2R)-2-dimethylamino-1-phenylpropyl-1]-ester
1,4-Dihydro-2,6-dimethyl-4-(3-nitrophenyl)-pyridin-3,5-dicarbonsäure-3-ethyl-5-[(1R),(2R)-2-dimethylamino-1-phenylpropyl-1]-ester
1,4-Dihydro-2,6-dimethyl-4-(3-nitrophenyl)-pyridin-3,5-dicarbonsäure-3-(2-methoxyethyl)-5-[(1R),(2R)-2-dimethylamino-1-phenylpropyl-1]-ester
1,4-Dihydro-2,6-dimethyl-4-(3-nitrophenyl)-pyridin-3,5-dicarbonsäure-3-(propyl-2)-5-[(1R),(2R)-2-dimethylamino-1-phenylpropyl-1]-ester

1,4-Dihydro-2,6-dimethyl-4-(3-nitrophenyl)-pyridin-3,5-dicarbonsäure-3-methyl-5-[(1S),(2S)-2-dimethylamino-1-phenylpropyl-1]-ester
1,4-Dihydro-2,6-dimethyl-4-(3-nitrophenyl)-pyridin-3,5-dicarbonsäure-3-ethyl-5-[(1S),(2S)-2-dimethylamino-1-phenylpropyl-1]-ester
1,4-Dihydro-2,6-dimethyl-4-(3-nitrophenyl)-pyridin-3,5-dicarbonsäure-3-(2-methoxyethyl)-5-[(1S),(2S)-2-dimethylamino-1-phenylpropyl-1]-ester
1,4-Dihydro-2,6-dimethyl-4-(3-nitrophenyl)-pyridin-3,5-dicarbonsäure-3-(propyl-2)-5-[(1S),(2S)-2-dimethylamino-1-phenylpropyl-1]-ester
4-(2,3-Dichlorphenyl)-1,4-dihydro-2,6-dimethyl-pyridin-3,5-dicarbonsäure-3-methyl-5-[(1R),(2R)-2-dimethylamino-1-phenylpropyl-1]-ester
4-(2,3-Dichlorphenyl)-1,4-dihydro-2,6-dimethyl-pyridin-3,5-dicarbonsäure-3-ethyl-5-[(1R),(2R)-2-dimethylamino-1-phenylpropyl-1]-ester
4-(2,3-Dichlorphenyl)-1,4-dihydro-2,6-dimethyl-pyridin-3,5-dicarbonsäure-3-(2-methoxyethyl)-5-[(1R),(2R)-2-dimethylamino-1-phenylpropyl-1]-ester
4-(2,3-Dichlorphenyl)-1,4-dihydro-2,6-dimethyl-pyridin-3,5-dicarbonsäure-3-(propyl-2)-5-[(1R),(2R)-2-dimethylamino-1-phenylpropyl-1]-ester
4-(2,3-Dichlorphenyl)-1,4-dihydro-2,6-dimethyl-pyridin-3,5-dicarbonsäure-3-methyl-5-[(1S),(2S)-2-dimethylamino-1-phenylpropyl-1]-ester
4-(2,3-Dichlorphenyl)-1,4-dihydro-2,6-dimethyl-pyridin-3,5-dicarbonsäure-3-ethyl-5-[(1S),(2S)-2-dimethylamino-1-phenylpropyl-1]-ester
4-(2,3-Dichlorphenyl)-1,4-dihydro-2,6-dimethyl-pyridin-3,5-dicarbonsäure-3-(2-methoxyethyl)-5-[(1S),(2S)-2-dimethylamino-1-phenylpropyl-1]-ester
4-(2,3-Dichlorphenyl)-1,4-dihydro-2,6-dimethyl-pyridin-3,5-dicarbonsäure-3-(propyl-2)-5-[(1S),(2S)-2-dimethylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1R),(2R)-2-dimethylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1S),(2S)-2-dimethylamino-1-phenylpropyl-1]-ester
1,4-Dihydro-2,6-dimethyl-4-(3-nitrophenyl)-pyridin-3,5-dicarbonsäure-3-methyl-5-[(1R),(2R)-2-methylamino-1-phenylpropyl-1]-ester
1,4-Dihydro-2,6-dimethyl-4-(3-nitrophenyl)-pyridin-3,5-dicarbonsäure-3-ethyl-5-[(1R),(2R)-2-methylamino-1-phenylpropyl-1]-ester
1,4-Dihydro-2,6-dimethyl-4-(3-nitrophenyl)-pyridin-3,5-dicarbonsäure-3-(2-methoxyethyl)-5-[(1R),(2R)-2-methylamino-1-phenylpropyl-1]-ester
1,4-Dihydro-2,6-dimethyl-4-(3-nitrophenyl)-pyridin-3,5-dicarbonsäure-3-(propyl-2)-5-[(1R),(2R)-2-methylamino-1-phenylpropyl-1]-ester
1,4-Dihydro-2,6-dimethyl-4-(3-nitrophenyl)-pyridin-3,5-dicarbonsäure-3-methyl-5-[(1S),(2S)-2-methylamino-1-phenylpropyl-1]-ester
1,4-Dihydro-2,6-dimethyl-4-(3-nitrophenyl)-pyridin-3,5-dicarbonsäure-3-ethyl-5-[(1S),(2S)-2-methylamino-1-phenylpropyl-1]-ester
1,4-Dihydro-2,6-dimethyl-4-(3-nitrophenyl)-pyridin-3,5-dicarbonsäure-3-(2-methoxyethyl)-5-[(1S),(2S)-2-methylamino-1-phenylpropyl-1]-ester
1,4-Dihydro-2,6-dimethyl-4-(3-nitrophenyl)-pyridin-3,5-dicarbonsäure-3-(propyl-2)-5-[(1S),(2S)-2-methylamino-1-phenylpropyl-1]-ester
4-(2,3-Dichlorphenyl)-1,4-dihydro-2,6-dimethyl-pyridin-3,5-dicarbonsäure-3-methyl-5-[(1R),(2R)-2-methylamino-1-phenylpropyl-1]-ester
4-(2,3-Dichlorphenyl)-1,4-dihydro-2,6-dimethyl-pyridin-3,5-dicarbonsäure-3-ethyl-5-[(1R),(2R)-2-methylamino-1-phenylpropyl-1]-ester
4-(2,3-Dichlorphenyl)-1,4-dihydro-2,6-dimethyl-pyridin-3,5-dicarbonsäure-3-(2-methoxyethyl)-5-[(1R),(2R)-2-methylamino-1-phenylpropyl-1]-ester
4-(2,3-Dichlorphenyl)-1,4-dihydro-2,6-dimethyl-pyridin-3,5-dicarbonsäure-3-(propyl-2)-5-[(1R),(2R)-2-methylamino-1-phenylpropyl-1]-ester
4-(2,3-Dichlorphenyl)-1,4-dihydro-2,6-dimethyl-pyridin-3,5-dicarbonsäure-3-methyl-5-[(1S),(2S)-2-methylamino-1-phenylpropyl-1]-ester
4-(2,3-Dichlorphenyl)-1,4-dihydro-2,6-dimethyl-pyridin-3,5-dicarbonsäure-3-ethyl-5-[(1S),(2S)-2-methylamino-1-phenylpropyl-1]-ester
4-(2,3-Dichlorphenyl)-1,4-dihydro-2,6-dimethyl-pyridin-3,5-dicarbonsäure-3-(2-methoxyethyl)-5-[(1S),(2S)-2-methylamino-1-phenylpropyl-1]-ester

4-(2,3-Dichlorphenyl)-1,4-dihydro-2,6-dimethyl-pyridin-3,5-dicarbonsäure-3-(propyl-2)-5-[(1S),(2S)-2-methylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1R),(2R)-2-methylamino-1-phenylpropyl-1]-ester
4-(2,1,3-Benzoxdiazol-4-yl)-1,4-dihydro-2,6-dimethyl-pyridin-3,5-dicarbonsäure-3-methyl-5-[(1S),(2S)-2-methylamino-1-phenylpropyl-1]-ester
1,4-Dihydro-2,6-dimethyl-4-(3-nitrophenyl)-pyridin-3,5-dicarbonsäure-3-methyl-5-[(1R),(2S)-2-(N-benzyl-N-methyl)-amino-1-phenylpropyl-1]-ester
1,4-Dihydro-2,6-dimethyl-4-(3-nitrophenyl)-pyridin-3,5-dicarbonsäure3-methyl-5-[(1S),(2R)-2-(N-benzyl-N-methyl)-amino-1-phenylpropyl-1]-ester
4-(2,3-Dichlorphenyl)-1,4-dihydro-2,6-dimethyl-pyridin-3,5-dicarbonsäure-3-methyl-5-[(1R),(2S)-2-(N-benzyl-N-methyl)-amino-1-phenylpropyl-1]-ester
4-(2,3-Dichlorphenyl)-1,4-dihydro-2,6-dimethyl-pyridin-3,5-dicarbonsäure-3-methyl-5-[(1S),(2R)-2-(N-benzyl-N-methyl)-amino-1-phenylpropyl-1]-ester
1,4-Dihydro-2,6-dimethyl-4-(3-nitrophenyl)-pyridin-3,5-dicarbonsäure-3-methyl-5-[(1R),(2R)-2-(N-benzyl-N-methyl)-amino-1-phenylpropyl-1]-ester
1,4-Dihydro-2,6-dimethyl-4-(3-nitrophenyl)-pyridin-3,5-dicarbonsäure3-methyl-5-[(1S),(2S)-2-(N-benzyl-N-methyl)-amino-1-phenylpropyl-1]-ester
4-(2,3-Dichlorphenyl)-1,4-dihydro-2,6-dimethyl-pyridin-3,5-dicarbonsäure-3-methyl-5-[(1R),(2R)-2-(N-benzyl-N-methyl)-amino-1-phenylpropyl-1]-ester
4-(2,3-Dichlorphenyl)-1,4-dihydro-2,6-dimethyl-pyridin-3,5-dicarbonsäure-3-methyl-5-[(1S),(2S)-2-(N-benzyl-N-methyl)-amino-1-phenylpropyl-1]-ester
und die Salze dieser Verbindungen.

Ein weiterer Gegenstand der Erfindung ist ein Verfahren zur Herstellung der erfindungsgemäßen Verbindungen und ihrer Salze. Das Verfahren ist dadurch gekennzeichnet, daß man

a) zur Herstellung von Verbindungen I, in denen R6 und/oder R7 eine andere Bedeutung als Wasserstoff haben, 1,4-Dihydropyridinderivate der Formel II

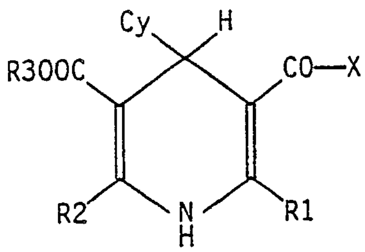

(II),

mit Ephedrinderivaten der Formel III

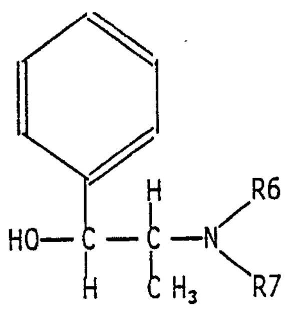

(III),

umsetzt, oder daß man

b) zur Herstellung von Verbindungen I, in denen R6 und/oder R7 eine andere Bedeutung als Wasserstoff haben, Aminocrotonsäurederivate der Formel IV

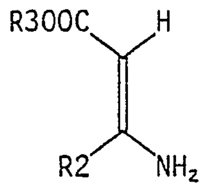

(IV)

mit Acrylsäurederivaten der Formel V

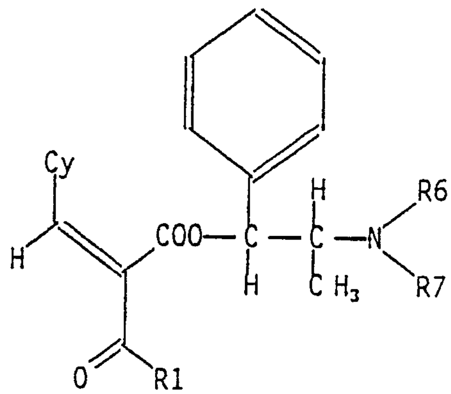

(V)

umsetzt, oder daß man

c) zur Herstellung von Verbindungen I, in denen R6 Wasserstoff bedeutet, aus Verbindungen der Formel I, in denen R6 eine in der Peptidchemie gebräuchliche N-Schutzgruppe darstellt, die Schutzgruppe abspaltet, oder daß man

d) zur Herstellung von Verbindungen I, in denen R6 und R7 eine andere Bedeutung als Wasserstoff haben, Verbindungen der Formel VI

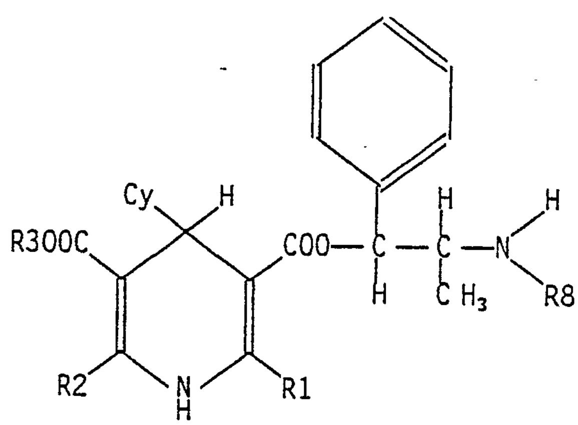

(VI)

worin R8 die Bedeutung von R6 oder R7 (mit Ausnahme der Bedeutung Wasserstoff) hat, mit Verbindungen der Formel VII

Z-R9 (VII)

worin R9 die Bedeutung von R7 oder R6 (mit Ausnahme der Bedeutung Wasserstoff) hat und Z eine Fluchtgruppe darstellt, umsetzt und gewünschtenfalls anschliessend an die Umsetzungen nach a, b, c oder d erhaltene Salze in die freien Basen oder erhaltene Basen in die Salze überführt, wobei Cy, R1, R2, R3, R4, R5, R6 und R7 (sofern nicht anders angegeben) die oben angegebenen Bedeutungen haben und X und Z Fluchtgruppen darstellen.

Ausgestaltungen des Verfahrens sind solche, bei denen in den Formeln II bis VII die Substituenten bzw. Symbole Cy, R1, R2, R3, R4, R5, R6 und R7 (sofern nicht anders angegeben) die in den Unter- und Nebenansprüchen angegebenen Bedeutungen haben und X und Z Fluchtgruppen darstellen.

Die Umsetzung von II mit III erfolgt in einer Weise, wie sie für die Herstellung von Estern bekannt ist. Je nach Art der Fluchtgruppe X, die vorzugsweise ein Halogenatom, insbesondere ein Chlor- oder Bromatom ist, kann die Reaktion gewünschtenfalls in Gegenwart einer Base (z.B. eines anorganischen Carbonates, wie Kaliumcarbonat, oder eines organischen Amins, wie Triethylamin) durchgeführt werden.

Das Verfahren wird in geeigneten, vorzugsweise inerten organischen Lösungsmitteln durchgeführt. Beispielsweise seien genannt Kohlenwasserstoffe, wie Toluol oder Xylol; Ether, wie Dioxan, Diethylether, Tetrahydrofuran oder Glykoldimethylether; oder Ketone, wie Aceton oder Ethylmethylketon; chlorierte Kohlenwasserstoffe, wie Methylenchlorid, Chloroform, Tetrachlorethylen oder Dichlorethan.

Die Reaktionstemperaturen können - je nach Reaktivität der Edukte - in einem weiten Bereich variieren. Im allgemeinen wird die Umsetzung bei Temperaturen zwischen 20° C und 150° C, vorzugsweise zwischen 20° C und 100° C durchgeführt.

Die Umsetzung gemäß Verfahrensvariante b) erfolgt in Analogie zur Hantz'schen Dihydropyridinsynthese, wie sie dem Fachmann geläufig ist.

Die Schutzgruppenabspaltung gemäß Verfahrensvariante c) erfolgt auf eine Weise, wie sie in der Peptidchemie gebräuchlich ist.

Die Umsetzung gemäß Verfahrensvariante d) erfolgt auf eine dem Fachmann geläufige Weise, wie sie für die Herstellung von tertiären Aminen bzw. von Amiden bekannt ist. Je nach Art der Fluchtgruppe Z, die insbesondere ein Halogenatom (Chlor, Brom oder Jod) ist, erfolgt die Umsetzung in Gegenwart einer Hilfsbase, z.B. eines anorganischen Carbonats, wie Kaliumcarbonat.

Die konfigurativ einheitlichen Verbindungen der Formel I und ihre Salze, die bevorzugter Gegenstand der Erfindung sind, erhält man beispielsweise ausgehend von konfigurativ einheitlichen Verbindungen II und III.

Die besonders bevorzugten Konfigurationsisomeren der Formel Ia erhält man vorteilhafterweise auch, indem man von racemischen Mischungen der Verbindungen II und konfigurativ einheitlichen Verbindungen III ausgeht und die beiden erhaltenen Diastereomeren auf übliche Weise (z.B. durch fraktionierte Kristallisation oder durch Chromatografie) trennt. Auch die nach Verfahrensvariante b erhaltenen Diastereomerengemische lassen sich - sofern von konfigurativ einheitlichen Verbindungen V ausgegangen wird - auf übliche Weise trennen.

Die konfigurativ einheitlichen 1,4-Dihydropyridinderivate II erhält man ausgehend von bekannten oder auf bekanntem Weg in analoger Weise herstellbaren enantiomer reinen N-geschützten 1,4-Dihydropyridincarbonsäuren [Chem. Pharm. Bull. 28, 2809 (1980)].

Die Isolierung und Reinigung der erhaltenen erfindungsgemäßen Substanzen erfolgt in an sich bekannter Weise z. B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen II, III, IV, V und VII sind literaturbekannt oder können in Analogie zu literaturbekannten Methoden hergestellt werden. Die Verbindungen VI erhält man beispielsweise gemäß Verfahrensvariante c).

Die erfindungsgemäßen Verbindungen der Formel I, in denen R6 eine Schutzgruppe bedeutet, sind auch wertvolle Zwischenprodukte, beispielsweise für die Herstellung von Verbindungen I mit R6 = H gemäß Verfahrensvariante c.

Die vorstehenden Herstellungsverfahren sind lediglich zur Verdeutlichung angegeben, und die Herstellung der erfindungsgemäßen Verbindungen der Formel I ist nicht auf dieses Verfahren beschränkt. Vielmehr ist auch jede Modifikation dieser Verfahren in gleicher Weise für die Herstellung der erfindungsgemäßen Verbindungen anwendbar.

Die folgenden Herstellungsbeispiele sollen die Erfindung näher erläutern, ohne sie einzuschränken. Schmp. bedeutet Schmelzpunkt, h steht für Stunden, Kp. steht für Siedepunkt, Zers. bedeutet Zersetzung.

## Beispiele

## Endprodukte

1. 1,4-Dihydro-2,6-dimethyl-4-(3-nitrophenyl)-pyridin-3,5-dicarbonsäure-3-ethyl-5-[(1R,2S)-2-dimethylamino-1-phenylpropyl-1]-ester-hydrochlorid

a) 3,90 g (±)-1,4-Dihydro-2,6-dimethyl-1-ethoximethyl-4-(3-nitrophenyl)-pyridin-3,5-dicarbonsäure-3-ethylester [Chem. Pharm. Bull. 27, 1426 (1979)] und 10 ml Oxalylchlorid werden bei Raumtemperatur solange gerührt, bis keine Gasentwicklung mehr stattfindet. Unter Zusatz von je 5 ml absolutem Toluol engt man den Ansatz im Wasserstrahlvakuum 3 x bis zur Gewichtskonstanz ein. Der dunkelbraune ölige Rückstand wird in 3 ml absolutem Dichlormethan aufgenommen und die Lösung zu einer Lösung von 1,79 g (1R,2S)-N-Methylephedrin und 1,4 ml Triethylamin in 15 ml absolutem Dichlormethan zugetropft. Nach Rühren über Nacht schüttelt man den Ansatz mit 50 ml Wasser und 3 x 50 ml Dichlormethan aus, trocknet die vereinigten organischen Phasen über Natriumsulfat und engt ein. Nach Reinigung des Rohproduktes durch Chromatographie über eine 3 x 30 cm Kieselgelsäule (Elutionsmittel: Dichlormethan + 2,5 % Methanol) überführt man das erhaltene Produkt mit etherischer HCl in das Hydrochlorid. Das eine Diastereomer der Titelverbindung kristallisiert aus einem Gemisch von Dichlormethan/Methanol/Diethylether in feinen Nadeln vom Schmp. 187 - 189 °C aus; $[\alpha]_D^{22}$ = + 283° (c=1, Methanol). Ausbeute: 1,4 g.

b) Die bei a) zurückbleibende Mutterlauge wird solange mit Diethylether versetzt, bis keine Kristalle mehr erhalten werden. Nach Einengen der letzten Mutterlauge erhält man einen fest aufschäumenden Rückstand, der in wenig Dichlormethan gelöst wird. Das weitere Diastereomer der Titelverbindung wird durch Eintropfen dieser Lösung in 1 l Petrolether/Diethylether (3 + 1) amorph ausgefällt. Ausbeute: 3,1 g; $[\alpha]_D^{22}$ = + 114,8° (c=1, Methanol); Schmp.: 137 - 150 °C (langsames Zerfließen).

2. 1,4-Dihydro-2,6-dimethyl-4-(3-nitrophenyl)-pyridin-3,5-dicarbonsäure-3-ethyl-5-[(1S,2R)-2-dimethylamino-1-phenylproply-1]-ester-hydrochlorid

a) Analog Beispiel 1 a) erhält man das eine Diastereomer der Titelverbindung, wenn das Säurechlorid mit 1,79 g (1S,2R)-N-Methylephedrin umgesetzt wird. Feine Nadeln vom Schmp. 188 - 190 °C; $[\alpha]_D^{22}$ = - 279,7° (c=1, Methanol); Ausbeute: 1,4 g.

b) Analog Beispiel 1 b) erhält man das weitere Diastereomer der Titelverbindung nach dem Ausfällen als feines amorphes Pulver vom Schmp.: 137 - 145 °C (langsames Zerfließen); $[\alpha]_D^{22}$ = - 118,1° (c=1, Methanol); Ausbeute: 2,6 g.

3. 1,4-Dihydro-2,6-dimethyl-4-(3-nitrophenyl)-pyridin-3,5-dicarbonsäure-3-methyl-5-[(1S, 2R)-2-(N-tert.-butoxicarbonyl-N-methyl)-amino-1-phenylpropyl-1]-ester

96 g 2-Acetyl-3-(3-nitrophenyl)-acrylsäure-[(1S,2R)-3-(N-tert.-butoxicarbonyl-N-methyl)-amino-1-phenylpropyl-1]-ester und 23 g 3-Amino-crotonsäuremethylester werden in 300 ml 2-Propanol 3 h unter Rückfluß zum Sieden erhitzt. Die abgekühlte Lösung wird eingeengt, der Rückstand in Essigsäureethylester aufgenommen und die Lösung mit 5 % Zitronensäure und 2 mal mit Wasser gewaschen. Nach dem Trocknen der organischen Phase über Natriumsulfat engt man ein und reinigt den Rückstand durch Chromatographie über eine kurze Kieselgelsäule mit Dichlormethan/Essigsäureethylester (95 + 5). Nach dem Einengen erhält man 20 g der fest aufgeschäumten Titelverbindung als Diastereomerengemisch; Schmp. 95 -105 °C (langsames Zerfließen).

4. 1,4-Dihydro-2,6-dimethyl-4-(3-nitrophenyl)-pyridin-3,5-dicarbonsäure-3-methyl-5-[(1S,2S)-2-(N-tert.-butoxicarbonyl-N-methyl)-amino-1-phenylpropyl-1]-ester

Analog der in Beispiel 3 beschriebenen Methode erhält man die Titelverbindung als Diastereomerengemisch aus dem entsprechenden Acrylsäureester und dem Crotonsäureester. Durch Umkristallisation des Diastereomerengemisches in Essigsäureethylester erhält man das 1. Diastereomer als feine Nadeln vom Schmp. 209 -210 °C, $[\alpha]_D^{22}$ = - 114,9° (c=1, Methanol), Rf = 0,3 (Dichlormethan/Essigsäureethylester 9 + 1) und aus der Mutterlauge das 2. Diastereomer als Nadeln vom Schmp. 188 - 189 °C, $[\alpha]_D^{22}$ = - 20,2° (c=1, Methanol), Rf = 0,25 (Dichlormethan/Essigsäureethylester 9 + 1).

5. 1,4-Dihydro-2,6-dimethyl-4-(3-nitrophenyl)-pyridin-3,5-dicarbonsäure-3-methyl-5-[(1R,2S)-2-(N-tert.-butoxicarbonyl-N-methyl)-amino-1-phenylpropyl-1]-ester

Analog der in Beispiel 3 beschriebenen Methode erhält man die Titelverbindung als Diastereomerengemisch aus dem entsprechenden Acrylsäureester und dem Crotonsäureester. Das Diastereomerengemisch hat einen Schmp. von 95 - 104 °C (langsames Zerfliessen, amorph ausgefällt in Diethylether/Petrolether 1 + 4). Durch mehrfache Umkristallistion des Diastereomerengemisches in Chloroform/Diisopropylether erhält man das 1. Diastereomer als feine Nadeln vom Schmp. 135 - 144 °C (langsames Zerfließen), oberer Fleck im DC (Kieselgel, Dichlormethan/Essigsäureethylester 9 + 1); $[\alpha]_D^{22}$ = + 267,0° (c = 1, Methanol).

6. 1,4-Dihydro-2,6-dimethyl-4-(3-nitrophenyl)-pyridin-3,5-dicarbonsäure-3-methyl-5-[(1R,2R)-2-(N-tert.-butoxicarbonyl-N-methyl)-amino-1-ohenylpropyl-1]-ester

Analog der in Beispiel 3 beschriebenen Methode erhält man die Titelverbindung als Diastereomerengemisch aus dem entsprechenden Acrylsäureester und dem Crotonsäureester. Durch mehrfaches Umkristallisieren des Diastereomerengemisches in Essigsäureethylester/Petrolether erhält man das 1. Diastereomer der Titelverbindung als feine Nadeln vom Schmp. 210 - 212 °C, $[\alpha]_D^{22}$ = + 112,7° (c=1, Methanol), oberer Fleck im DC (Kieselgel, Dichlormethan/Essigsäureethylester 9 + 1). Aus den Mutterlaugen erhält man nach mehrfachem Umkristallisieren das 2. Diastereomer als feine Nadeln vom Schmp. 198 - 200 °C, $[\alpha]_D^{22}$ = + 11,2° (c=1, Methanol), unterer Fleck im DC.

7. 1,4-Dihydro-2,6-dimethyl-4-(3-nitrophenyl)-pyridin-3,5-dicarbonsäure-3-methyl-5-[(1S,2R)-2-methylamino-1-phenylpropyl-1]-ester-hydrochlorid

75 g 1,4-Dihydro-2,6-dimethyl-4-(3-nitrophenyl)-pyridin-3,5-dicarbonsäure-3-methyl-5-[(1S,2R)-2-(N-tert.-butoxicarbonyl-N-methyl)-amino-1-phenylpropyl-1]-ester (Beispiel 3) werden in 500 ml Ethanol und 21 ml konzentrierter Salzsäure (d = 1,19) bei 60 °C 4 h gerührt. Nach dem Einengen der Lösung extrahiert man den Rückstand mit Wasser/Dichlormethan, trocknet die oranische Phase über Natriumsulfat, und engt ein. Aus dem erhaltene fest aufgeschäumten Rückstand erhält man nach Lösen in Methanol, Versetzen mit Diethylether bis zur ersten bleibenden feinen Trübung und Anreiben der Lösung ein Kristallisat, das erneut 3 x in Methanol/Diethylether umkristallisiert wird. Man erhält 18 g des einen Diastereomers als feine Nadeln vom Schmp. 229 - 230 °C; $[\alpha]_D^{22}$ = - 88° (c=1, Methanol); Rf = 0,7 (Kieselgel, Laufmittel Dichlormethan/Ethanol/Triethylamin = 17,5/2/0,5).

Aus den vereinigten Mutterlaugen erhält man analog aus Methanol und Diethylether nach mehrfachem Umkristallisieren 30 g des anderen Diastereomers als feine Nadeln vom Schm. 218 - 219 °C; $[\alpha]_D^{22}$ = - 201° (c=1, Methanol) Rf = 0,75 (Kieselgel, Laufmittel Dichlormethan/Ethanol/Triethylamin = 17,5/2/0,5).

8. 1,4-Dihydro-2,6-dimethyl-4-(3-nitrophenyl)-pyridin-3,5-dicarbonsäure-3-methyl-5-[(1R,2S)-2-methylamino-1-phenylpropyl-1]-ester-hydrochlorid

Analog Beispiel 7 erhält man aus 225 g 1,4-Dihydro-2,6-dimethyl-4-(3-nitrophenyl)-pyridin-3,5-dicarbonsäure-3-methyl-5-[(1R,2S)-2-(N-tert.-butoxicarbonyl-N-methyl)-amino-1-phenylpropyl-1]-ester (Beispiel 5) nach Abspalten der Schutzgruppe 29 g des einen Diasteromers als feine Nadeln vom Schmp. 229 - 230 °C; $[\alpha]_D^{22}$ = + 88,8° (c=1, Methanol); Rf = 0,5 und 42 g des anderen Diastereomers als Nadeln vom Schmp. 219 - 220 °C; $[\alpha]_D^{22}$ = + 202,6° (c=1, Methanol), Rf = 0,55. Das L-(+)-Tartrat dieses Diastereomers hat den Schmp. 195,5 - 198,5 °C (Zersetzung), $[\alpha]_D^{22}$ = + 171,0° (c = 1, Methanol).

9. 1,4-Dihydro-2,6-dimethyl-4-(3-nitrophenyl)-pyridin-3,5-dicarbonsäure-3-methyl-5-[(1S,2S)-2-methylamino-1-phenylpropyl-1]-ester-hydrochlorid

Analog Beispiel 7 erhält man aus 200 g 1,4-Dihydro-2,6-dimethyl-4-(3-nitrophenyl)-pyridin-3,5-dicarbonsäure-3-methyl-5-[(1S,2S)-2-(N-tert.-butoxicarbonyl-N-methyl)-amino-1-phenylpropyl-1]-ester (Beispiel 4) nach Abspaltung der Schutzgruppe 55 g des einen Diastereomers als feine Nadeln vom Schmp. 208 - 209 °C; $[\alpha]_D^{22}$ = - 162,8° (c = 1, Methanol), Rf = 0,65, und 24 g weiteres Diastereomer als grobe Kristalle vom Schmp. 173 - 174 °C, $[\alpha]_D^{22}$ = - 66,3° (c = 1, Methanol), Rf = 0,60.

10. 1,4-Dihydro-2,6-dimethyl-4-(3-nitrophenyl)-pyridin-3,5-dicarbonsäure-3-methyl-5-[(1R,2R)-2-methylamino-1-phenylpropyl-1]-ester-hydrochlorid

Analog Beispiel 7 erhält man aus 66 g 1,4-Dihydro-2,6-dimethyl-4-(3-nitrophenyl)-pyridin-3,5-dicarbonsäure-3-methyl-5-[(1R,2R)-2-(N-tert.-butoxicarbonyl-N-methyl)-amino-1-phenylpropyl-1]-ester (1. Diastereomer des Beispiels 6) nach Abspaltung der Schutzgruppe 52 g des einen Diastereomers der Titelverbindung als feine Nadeln vom Schmp. 211 - 213 °C, $[\alpha]_D^{22}$ = + 165,6° (c = 1, Methanol), und aus 22 g des 2. Diastereomers des Beispiels 6 nach Entschützung 17 g des anderen Diastereomers der Titelverbindung als Nadeln vom Schmp. 172 -174 °C, $[\alpha]_D^{22}$ = + 68,2° (c = 1, Methanol).

11. 1,4-Dihydro-2,6-dimethyl-4-(3-nitrophenyl)-pyridin-3,5-dicarbonsäure-3-methyl-5-[(1R,2S)-2-methyl-2-benzylamino-1-phenylpropyl-1]-ester-hydrochlorid

2,58 g des in Beispiel 8 erhaltenen Diastereomers mit $[\alpha]_D^{22}$ = + 202,6° und 0,8 ml Benzylbromid werden mit 3 g gepulvertem Kaliumcarbonat in 30 ml Dioxan/n-Butanol (1 + 1) 4 h unter Rückfluß zum Sieden erhitzt. Nach Absaugen des Niederschlages engt man das Filtrat ein, nimmt den Rückstand in Dichlormethan auf und filtriert die Lösung über eine 5 cm dicke Kieselgelschicht. Nach dem Einengen der Produktlösung auf ein kleines Volumen extrahiert man die hellgelbe Lösung mit 50 ml 1 N Salzsäure und wäscht noch 2 mal mit je 50 ml Wasser. Nach dem Trocknen der organischen Phase über Natriumsulfat engt man ein und kristallisiert den fest aufgeschäumten Rückstand in Essigsäureethylester/Diisopropylether aus. Schmp. 223 - 224 °C,$[\alpha]_D^{22}$ = + 244,4° (c = 1, Methanol); Ausbeute; 2,6 g.

12. 1,4-Dihydro-2,6-dimethyl-4-(3-nitrophenyl)-pyridin-3,5-dicarbonsäure-3-methyl-5-[(1R,2S)-2-(N-ethyl-N-methyl)-amino-1-phenylpropyl-1]-ester-hydrochlorid

Analog Beispiel 11 erhält man die Titelverbindung aus dem Diastereomer des Beispiels 8 mit $[\alpha]_D^{22}$ = + 202,6° und Ethyljodid als feine Nadeln vom Schmp. 219 - 220 °C(aus Methanol/Diethylether), $[\alpha]_D^{22}$ = + 305,3° (c = 1, Methanol).

13. 1,4-Dihydro-2,6-dimethyl-4-(3-nitrophenyl)-pyridin-3,5-dicarbonsäure-3-methyl-5-[(1R,2S)-2-(N-methyl)-acetamido-1-phenylpropyl-1]-ester

2,58 g des in Beispiel 8 erhaltenen Diasteromers mit $[\alpha]_D^{22}$ = + 202,6° werden in 25 ml absolutem Dichlormethan und 1,6 ml Triethylamin gelöst und zur Lösung langsam 0,44 ml Acetylchlorid in 5 ml absolutem Dichlormethan zugetropft. Nach 2 h Rühren bei Raumtemperatur nimmt man den Ansatz in 100 ml Dichlormethan auf und wäscht 2 mal mit je 100 ml Wasser. Nach dem Trocknen der organischen Phase über Natriumsulfat und Einengen fällt man die Titelverbindung amorph in Petrolether aus; Schmp. 100 - 112 °C (langsames Zerfließen), $[\alpha]_D^{22}$ = + 299,4° (c = 1, Methanol), Ausbeute: 2,5 g.

14. 1,4-Dihydro-2,6-dimethyl-4-(3-nitrophenyl)-pyridin-3,5-dicarbonsäure-3-methyl-5-[(1R,2S)-2-(N-methyl-N-3-phenylprop-2-enyl)-amino-1-phenylpropyl-1]-ester-hydrochlorid

Analog Beispiel 11 erhält man die Titelverbindung aus dem Diasteromer des Beispiels 8 mit $[\alpha]_D^{22}$ = + 202,6° und Cinnamylbromid als amorph ausgefälltes feines Pulver vom Schmp. 142 - 150 °C (langsames

Zerfließen), $[\alpha]_D^{22}$ = + 218,3° (c = 1, Methanol).

15. 1,4-Dihydro-2,6-dimethyl-4-(3-nitrophenyl)-pyridin-3,5-dicarbonsäure-3-methyl-5-[(1R,2R)-2-(N-3,4,5-trimethoxybenzyl)-methyl-amino-1-phenylpropyl-1]-ester-hydrochlorid

Analog Beispiel 11 erhält man die Titelverbindung aus dem Diastereomer des Beispiels 10 mit $[\alpha]_D^{22}$ = + 165,6° und 3,4,5-Trimethoxybenzylchlorid als amorph ausgefälltes feines Pulver vom Schmp. 145 - 150°C (langsames Zerfliessen); $[\alpha]_D^{22}$ = + 145,2° (c = 1, Methanol).

16. 1,4-Dihydro-2,6-dimethyl-4-(3-nitrophenyl)-pyridin-3,5-dicarbonsäure-3-methyl-5-[(1R,2S)-2-amino-1-phenylpropyl-1]-ester-hydrochlorid

Analog Beispiel 7 erhält man das eine Diastereomer der Titelverbindung als feine Nadeln mit dem Schmp. 230,5 - 231,5°C (Zersetzung), $[\alpha]_D^{22}$ = + 216,4° (c = 1, Methanol) und das andere Diastereomer als amorph ausgefälltes feines Pulver vom Schmp. 165 - 180°C (langsames Zerfließen), $[\alpha]_D^{22}$ = + 104,1° (c = 1, Methanol) nach Abspaltung der N-tert.-Butoxicarbonyl-Schutzgruppe aus dem nach Verfahren Beispiel 3 analog hergestellten Diasteromerengemisch der (1R,2S)-2-Amino-1-phenylpropylderivate.

17. 1,4-Dihydro-2,6-dimethyl-4-(R)-(3-nitrophenyl)-pyridin-3,5-dicarbonsäure-3-methyl-5-[(1S,2R)-2-amino-1-phenylpropyl-1]-ester-hydrochlorid

Analog Beispiel 16 erhält man das eine Diastereomer der Titelverbindung als grobe würfelige Kristalle vom Schmp. 235 - 237°C (Zersetzung), $[\alpha]_D^{22}$ = - 218,6° ( c = 1, Methanol) und das andere Diastereomer als amorph ausgefälltes feines Pulver vom Schmp. 158 - 173°C (langsames Zerfliessen), $[\alpha]_D^{22}$ = - 104,3° (c = 1, Methanol) aus den entsprechenden (1S,2R)-2-Amino-1-phenylpropyl-Derivaten.

18. 1,4-Dihydro-2,6-dimethyl-4-(3-nitrophenyl)-pyridin-3,5-dicarbonsäure-3-methyl-5-[(1R,2S)-2-(3,4,5-trimethoxibenzylamino)-1-phenylpropyl-1]-ester-hydrochlorid

Analog Beispiel 11 erhält man die Titelverbindung aus dem Diastereomer des Beispiels 16 mit $[\alpha]_D^{22}$ = + 216,4° und 3,4,5-Trimethoxybenylchlorid als feine Nadeln vom Schmp. 214 - 215°C, $[\alpha]_D^{22}$ = + 154,6° (c = 1, Methanol).

19. 1,4-Dihydro-2,6-dimethyl-4-(3-nitrophenyl)-pyridin-3,5-dicarbonsäure-3-methyl-5-[(1R,2S)-2-(N-2-methoxy-5-nitrobenzyl)-methylamino-1-phenylpropyl-1]-ester-hydrochlorid

Analog Beispiel 11 erhält man die Titelverbindung aus dem Diastereomer des Beispiels 8 mit $[\alpha]_D^{22}$ = + 202,6° und 2-Methoxy-5-nitrobenzylbromid als feine Kristalle vom Schmp. 156 - 167°C (langsames Zerfließen unter Zersetzung), $[\alpha]_D^{22}$ = + 197,5° (c = 1, Methanol).

20. 1,4-Dihydro-2,6-dimethyl-4-(3-nitrophenyl)-pyridin-3,5-dicarbonsäure-3-methyl-5-[(1R,2S)-2-dimethylamino-1-phenylpropyl-1]-ester-hydrochlorid

Analog Beispiel 1 a) erhält man das eine Diastereomer der Titelverbindung, wenn das Säurechlorid des (±)-1,4-Dihydro-2,6-dimethyl-4-(3-nitrophenyl)-pyridin-3,5-dicarbonsäure-3-methylesters mit (1R,2S)-2-Dimethylamino-1-phenylpropanol umgesetzt wird, als feine Nadeln vom Schmp. 203 - 204°C (Zersetzung), $[\alpha]_D^{22}$ = + 297,7° (c = 1, Methanol).

## Ausgangsverbindungen

2-Acetyl-3-(3-nitrophenyl)-acrylsäure-[2-(N-tert.-butoxicarbonyl-N-methyl)-amino-1-phenylpropyl-1]-ester

Durch Umsetzung von 3-Nitrobenzaldehyd mit Acetessigsäure-[2-(N-tert.-butoxicarbonyl-N-methyl)-amino-1-phenylpropyl-1)-ester unter azeotropen Kondensationsbedingungen erhält man die Titelverbindung in der in der Europäischen Patentanmeldung 176 956 beschriebenen Weise. Die Ausgangsverbindung Acetessigsäure-[2-(N-tert.-butoxicarbonyl-N-methyl)-amino-1-phenylpropyl-1]-ester erhält man ebenfalls nach dem in der Europäischen Patentanmeldung beschriebenen Verfahren durch Umsetzung von N-tert.-Butoxicarbonylephedrin mit Diketen. N-tert. Butoxicarbonylephedrin erhält man durch das dem Fachmann aus der Aminosäure-Schutzgruppenchemie bekannte Verfahren zur Herstellung von N-tert.-Butoxicarbonyl-geschützten Aminosäuren.

## Gewerbliche Anwendbarkeit

Die erfindungsgemäßen Verbindungen der Formel I und ihre Salze besitzen wertvolle Eigenschaften, die sie gewerblich verwertbar machen. Sie stellen insbesondere wirksame Vasodilatoren mit coronartherapeutischen Eigenschaften dar. Die pharmakologische Wirksamkeit der erfindungsgemäßen Verbindungen, die gepaart ist mit einer geringen Toxizität, zeigt sich insbesondere in einer rasch eintretenden, starken und optimal anhaltenden Blutdrucksenkung nach oraler Applikation. Darüberhinaus besitzen die erfindungsgemäßen Verbindungen hemmende Wirkung auf den Calciumeinstrom sowie fördernde Wirkung auf den Kaliumausstrom von Zellen, glattmuskulär relaxierende und peripher, coronar, cerebral und renal gefäßerweiternde sowie salidiuretische, antithrombotische, antiarteriosklerotische und günstige hämorheologische Eigenschaften.

In ihrer ausgezeichneten Wirksamkeit, die gepaart ist mit einer geringen Toxizität und dem Fehlen wesentlicher Nebenwirkungen, unterscheiden sich die erfindungsgemäßen Verbindungen in überraschender und vorteilhafter Weise von den Verbindungen des Standes der Technik.

Als vorteilhafte Eigenschaften der Verbindungen I sind beispielsweise zu nennen: das Ausmaß der Blutdrucksenkung, das optimale Anhalten der Blutdrucksenkung, die gute Steuerbarkeit der Blutdrucksenkung, die überraschend geringe Herzfrequenzsteigerung, die ausgezeichnete Bioverfügbarkeit, die große therapeutische Breite, das Fehlen kinetischer Interaktionen mit anderen Substanzen, das Ausbleiben einer Toleranzentwicklung, die ausgewogenen physikalischen Eigenschaften, die große Stabilität und insbesondere die überraschend gute Wasserlöslichkeit.

Die ausgezeichnete Wirksamkeit der erfindungsgemäßen Verbindungen der Formel I und ihrer Salze gestattet ihren Einsatz in der Humanmedizin, wobei als Indikation insbesondere primäre (essentielle) und sekundäre, arterielle und pulmonale Hypertonien aller Schweregrade, koronare Herzkrankheiten (Koronarinsuffizienz, Angina Pectoris, Myocardinfarkt etc.), periphere und cerebrale Zirkulationsstörungen (Gehirnschlag, temporäre cerebrale Durchblutungsstörungen, Migräne, Schwindel, renale Arterienverengung etc.), hypertrophe Kardiomyopathie, Herzin suffizienz, Krankheiten, die auf einer erhöhten Wasser- und Natriumretention beruhen und Krankheiten, die auf einem erhöhten Calciumeinstrom beruhen, wie z.B. Spasmen glattmuskulärer Organe (Atemwege, Gastrointestinaltrakt, Urogenitaltrakt etc.) sowie Arrhythmie, Arteriosklerose und Zellschädigungen verschiedener Genese (z.B. Hypoxie) in Betracht kommen.

Ein weiterer Gegenstand der Erfindung ist daher ein Verfahren zur Behandlung von Säugetieren, insbesondere Menschen, die an einer der obengenannten Krankheiten erkrankt sind. Das Verfahren ist dadurch gekennzeichnet, daß man dem erkrankten Individuum eine therapeutisch wirksame und pharmakologisch verträgliche Menge einer oder mehrerer Verbindungen der Formel I verabreicht.

Gegenstand der Erfindung sind außerdem die Verbindungen der Formel I zur Anwendung bei der Behandlung der genannten Krankheiten.

Ebenso umfaßt die Erfindung die Verwendung von Verbindungen der Formel I bei der Herstellung von Arzneimitteln, die zur Bekämpfung der genannten Krankheiten eingesetzt werden.

Ein weiterer Gegenstand der Erfindung sind Arzneimittel, die eine oder mehrere Verbindungen der allgemeinen Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragees, Kapseln, Suppositorien, Pflastern (z.B. als TTS), Emulsionen, Suspensionen, Aerosolen, Sprays, Salben, Cremes, Gelen oder Lösungen eingesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten, Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, rektal, per inhalationem oder parenteral (insbesondere perlingual, intravenös oder percutan) appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 10, vorzugsweise 0,05 bis 5 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Bei einschleichender Dosierung wird zu Beginn der Behandlung eine geringere Dosis verabreicht, dann langsam auf eine höhere Dosis übergegangen. Nach Erreichen des gewünschten Therapieerfolges wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der genannten Krankheiten eingesetzt werden, so können die pharmazeutischen Zubereitungen auch einen oder mehrere andere pharmakologisch aktive Bestandteile anderer Arzneimittelgruppen, wie andere Vasodilatoren, Antihypertensiva, alpha-1-Rezeptorenblocker, alpha-2-Rezeptorstimulatoren, beta-1-Rezeptorenblokker, beta-2-Rezeptorstimulatoren, ACE-Hemmstoffe, Nitroverbindungen, Cardiotonika, Diuretika, Saluretika, Alkaloide, Analgetika, Lipidsenker, Antikoagulantien, Anticholinergika, Methylxanthine, Antiarrhythmika, Antihistaminika, Dopaminstimulatoren, Serotonin-Rezeptorenblocker etc., wie Nifedipin, Dihydralazin, Prazosin, Clonidin, Atenolol, Labetalol, Fenoterol, Captopril, Isosorbiddinitrat, Digoxin, Milrinon, Mefrusid, Clopamid, Spironolacton, Chlorthalidon, Furosemid, Polythiazid, Hydrochlorothiazid, Reserpin, Dihydroergocristin, Rescinnamin, Rauwolfia-Gesamtalkaloide, Acetylsalicylsäure, Bezafibrat, Warfarin, Atropin, Theophyllin, Lidocain, Astemizol, Bromocryptin, Ketanserin etc. enthalten.

## Pharmakologie

Die antihypertensive Wirksamkeit der erfindungsgemäßen Verbindungen kann am Modell der spontan hypertonen Ratte nachgewiesen werden.

Zur Bestimmung der antihypertensiven Wirkung werden die unten aufgeführten Verbindungen in den angegebenen Dosen an zwei aufeinander folgenden Tagen an je 6 männlichen Ratten (Stamm SHR/N/Ibm/Bm, 250-350 g) mit genetisch bedingtem Hochdruck (systolischer Blutdruck > 180 mmHg) täglich einmal mittels Schlundsonde verabfolgt. Die Messung des Blutdrucks erfolgt jeweils 2, 6 und 24 Stunden nach Substanzgabe.

Die Blutdruckmessung wird in einer Wärmekammer bei 36 °C vorgenommen, um eine bessere Durchblutung der Schwanzarterie zu erreichen. Hierzu werden die Tiere in perforierte Lochblechkäfige verbracht und 20 - 40 Min. nach Beginn der Aufwärmung gemessen. Zur Messung des systolischen arteriellen Drucks wird eine ringförmige Manschette mit aufblasbarer Gummimembran zur Unterbindung der Durchblutung und ein ringförmiger Piezokristallaufnehmer zur Erfassung der Pulswellen auf den Schwanz aufgeschoben. Nach erfolgter Unterbindung des Blutstroms in der Schwanzarterie wird der Manschettendruck kontinuierlich reduziert. Die Wiederkehr der Pulswellen bei Druckablassen wird automatisch als systolischer Blutdruck erkannt und ausgedruckt (Bühler, R. et al.: Microprocessor-based automation of blood pressure measurement in the conscious rat. Proceedings of the 4th international symposium on rats with spontaneous hypertension and related studies, Rascher, R. et al. (Eds.), Schattauer Verlag, Stuttgart, New York, 1982, S. 410-413). Pulssignale und Druckverlauf werden zur Auswertung graphisch aufgezeichnet.

Zur Gewöhnung an den Meßvorgang werden die Tiere vor Substanzprüfung 14 Tage trainiert. In der zweiten Trainingswoche werden Blutdruck-Vorwerte erhoben. Tiergruppen, die Substanz erhalten, werden gegen eine Kontrollgruppe geprüft. In der anschließenden Tabelle werden die untersuchten Verbindungen durch laufende Nummern gekennzeichnet, die mit den jeweiligen Beispielnummern übereinstimmen. Bei getrennten Diastereomeren ist der Drehwert des untersuchten Diastereomers angegeben.

Tabelle I gibt für die Vertreter der erfindungsgemäßen Verbindungen die prozentuale Senkung des

Blutdrucks (BP) nach oraler Verabreichung bei der Ratte wieder.

Tabelle I

%-Änderungen (BP) an genetisch hypertonen Ratten nach täglich einmaliger p.o.-Applikation an zwei aufeinanderfolgenden Tagen (N = 6/Dosis).

| Beisp. Nr. | Dosis µmol/kg | BP (% Änderung vs. Kontrolle), Mittelwert für Meßzeitpunkte: Stunden nach Gabe (1. und 2. Tag) | | |
|---|---|---|---|---|
| | | 2h | 6h | 24h |
| 1a | 25 | -47,5 | -44,5 | - 8,5 |
| 1b | 25 | -40,0 | -30,3 | - 6,0 |
| 2b | 25 | -40,0 | -34,3 | - 4,5 |
| 7 (-88°) | 25 | -56,0 | -46,0 | -11,5 |
| 8 (+202,6°) | 25 | -55,0 | -43,0 | -10,5 |
| 9 (-66,3°) | 25 | -55,0 | -49,5 | - 2,0 |
| 10 (+165,5°) | 25 | -52,0 | -39,5 | + 6,5 |
| 11 | 25 | -53,5 | -48,0 | - 8,5 |
| 12 | 25 | -48,5 | -38,0 | - 8,5 |
| 14 | 25 | -51,5 | -41,5 | - 9,0 |
| 15 | 25 | -62,5 | -56,0 | -37 |
| 16 (+216,4°) | 25 | -59,0 | -56,5 | -21,5 |
| 17 (-104,3°) | 25 | -56,0 | -47,0 | -14,5 |
| 19 | 10 | -42,0 | -23,5 | -10,5 |

## Ansprüche

1. Verbindungen der Formel I

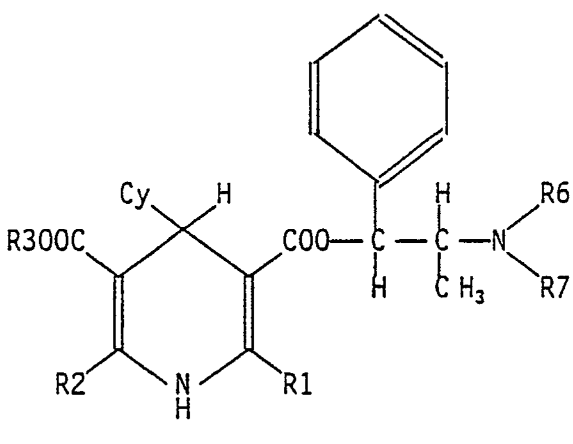

(I)

worin Cy einen Cyclus der Formel

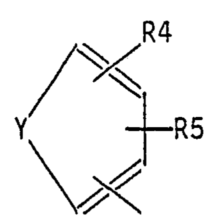

darstellt, in dem Y Sauerstoff (O), Schwefel (S), Vinylen (-CH = CH-), Azomethin (-CH = N-) oder eine Gruppe der Formel

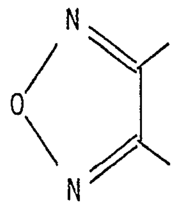

oder

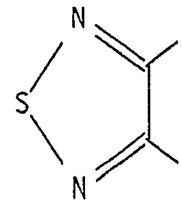

bedeutet,

R1 1-6C-Alkyl oder 3-7C-Alkoxyalkyl bedeutet,

R2 1-6C-Alkyl oder 3-7C-Alkoxyalkyl bedeutet,

R3 Wasserstoff, 1-6C-Alkyl oder 3-7C-Alkoxyalkyl bedeutet,

R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino, Mono- oder Di-1-4C-alkylamino oder gemeinsam Methylendioxy bedeuten,

R6 Wasserstoff, 1-6C-Alkyl, 1-4C-Alkoxycarbonyl, 2-5C-Acyl, Arylcarbonyl, Furoyl oder eine in der Peptidchemie gebräuchliche N-Schutzgruppe bedeutet und

R7 Wasserstoff, 1-6C-Alkyl, Aryl-1-4C-alkyl, Aryl-2-4C-alkenyl oder Aryl-2-4C-alkinyl bedeutet, wobei

Aryl für Phenyl, substituiertes Phenyl mit einem oder zwei Substituenten aus der Gruppe Halogen, 1-4C-Alkyl,1-4C-Alkoxy, Hydroxy oder Nitro oder substituiertes Phenyl mit einem, zwei oder drei Substituenten aus der Gruppe 1-4C-Alkoxy steht,

und die Salze dieser Verbindungen.

2. Verbindungen der Formel I nach Anspruch 1, worin

Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl, 2,3-Methylendioxyphenyl oder 2,1,3-Benzoxdiazol-4-yl bedeutet,

R1 Methyl, Ethyl oder Methoxyethyl bedeutet,

R2 Methyl, Ethyl oder Methoxyethyl bedeutet,

R3 Methyl, Ethyl oder Methoxyethyl bedeutet,

R6 Wasserstoff, Methyl, Ethyl, tert.-Butoxycarbonyl oder Acetyl bedeutet und

R7 Wasserstoff, Methyl, Benzyl, Chlorbenzyl, Brombenzyl, Methoxybenzyl, Dimethoxybenzyl, Trimethoxybenzyl, Hydroxybenzyl, Methylbenzyl, Dimethylbenzyl, Nitrobenzyl, Methoxy-nitrobenzyl oder 3-Phenyl-2-propenyl bedeutet,

und die Salze dieser Verbindungen.

3. Verbindungen der Formel I nach Anspruch 1, worin

Cy 3-Nitrophenyl, 2-Chlorphenyl, 2,3-Dichlorphenyl, 2-Trifluormethylphenyl oder 2,1,3-Benzoxdiazol-4-yl bedeutet,

R1 Methyl oder Ethyl bedeutet,

R2 Methyl oder Ethyl bedeutet,

R3 Methyl, Ethyl oder Methoxyethyl bedeutet,

R6 Wasserstoff, Methyl, Ethyl, tert.-Butoxycarbonyl oder Acetyl bedeutet und

R7 Wasserstoff, Methyl, Benzyl, 3,4,5-Trimethoxybenzyl, 2-Methoxy-5-nitrobenzyl oder 3-Phenyl-2-propenyl bedeutet,

und ihre Salze.

4. Verbindungen der Formel I nach Anspruch 1, worin

Cy 3-Nitrophenyl bedeutet,

R1 Methyl bedeutet,

R2 Methyl bedeutet,

R3 Methyl oder Ethyl bedeutet,

R6 Wasserstoff, Methyl oder Ethyl bedeutet und

R7 Methyl, Benzyl, 3,4,5-Trimethoxybenzyl, 2-Methoxy-5-nitrobenzyl oder 3-Phenyl-2-propenyl bedeutet,

und ihre Salze.

5. Verbindungen der Formel I nach Anspruch 1, worin
Cy 3-Nitrophenyl bedeutet,
R1 Methyl bedeutet,
R2 Methyl bedeutet,
R3 Methyl oder Ethyl bedeutet,
R6 Wasserstoff oder Methyl bedeutet und
R7 Methyl oder Benzyl bedeutet,
und ihre Salze.

6. Verbindungen der Formel I nach Anspruch 1, worin
Cy 3-Nitrophenyl,
R1 Methyl,
R2 Methyl,
R3 Methyl,
R6 Wasserstoff und
R7 Methyl bedeutet,
und ihre Salze.

7. Verbindung ausgewählt aus der Gruppe bestehend aus
1,4-Dihydro-2,6-dimethyl-4-(3-nitrophenyl)-pyridin-3,5-dicarbonsäure-3-ethyl-5-[(1R,2S)-2-dimethylamino-1-phenylpropyl-1]-ester
1,4-Dihydro-2,6-dimethyl-4-(3-nitrophenyl)-pyridin-3,5-dicarbonsäure-3-ethyl-5-[(1S,2R)-2-dimethylamino-1-phenylproplyl-1]-ester
1,4-Dihydro-2,6-dimethyl-4-(3-nitrophenyl)-pyridin-3,5-dicarbonsäure-3-methyl-5-[(1S,2R)-2-methylamino-1-phenylpropyl-1]-ester
1,4-Dihydro-2,6-dimethyl-4-(3-nitrophenyl)-pyridin-3,5-dicarbonsäure-3-methyl-5-[(1R,2S)-2-methylamino-1-phenylpropyl-1]-ester
1,4-Dihydro-2,6-dimethyl-4-(3-nitrophenyl)-pyridin-3,5-dicarbonsäure-3-methyl-5-[(1S,2S)-2-methylamino-1-phenylpropyl-1]-ester
1,4-Dihydro-2,6-dimethyl-4-(3-nitrophenyl)-pyridin-3,5-dicarbonsäure-3-methyl-5-[(1R,2R)-2-methylamino-1-phenylpropyl-1]-ester
1,4-Dihydro-2,6-dimethyl-4-(3-nitrophenyl)-pyridin-3,5-dicarbonsäure-3-methyl-5-[(1R,2S)-2-methyl-2-benzylamino-1-phenylpropyl-1]-ester
1,4-Dihydro-2,6-dimethyl-4-(3-nitrophenyl)-pyridin-3,5-dicarbonsäure-3-methyl-5-[(1R,2S)-2-(N-ethyl-N-methyl)-amino-1-phenylpropyl-1]-ester
1,4-Dihydro-2,6-dimethyl-4-(3-nitrophenyl)-pyridin-3,5-dicarbonsäure-3-methyl-5-[(1R,2S)-2-(N-methyl-N-3-phenylprop-2-enyl)-amino-1-phenylpropyl-1]-ester
1,4-Dihydro-2,6-dimethyl-4-(3-nitrophenyl)-pyridin-3,5-dicarbonsäure-3-methyl-5-[(1R,2R)-2-(N-3,4,5-trimethoxybenzyl)-methyl-amino-1-phenylpropyl-1]-ester
1,4-Dihydro-2,6-dimethyl-4-(3-nitrophenyl)-pyridin-3,5-dicarbonsäure-3-methyl-5-[(1R,2S)-2-(3,4,5-trimethoxibenzylamino)-1-phenylpropyl-1]-ester
1,4-Dihydro-2,6-dimethyl-4-(3-nitrophenyl)-pyridin-3,5-dicarbonsäure-3-methyl-5-[(1R,2S)-2-(N-2-methoxy-5-nitrobenzyl)-methylamino-1-phenylpropyl-1]-ester
1,4-Dihydro-2,6-dimethyl-4-(3-nitrophenyl)-pyridin-3,5-dicarbonsäure-3-methyl-5-[(1R,2S)-2-dimethylamino-1-phenylpropyl-1]-ester
und den Salzen dieser Verbindungen.

8. Verfahren zur Herstellung der Verbindungen der Formel I nach Anspruch 1 und ihrer Salze, dadurch gekennzeichnet, daß man

a) zur Herstellung von Verbindungen I, in denen R6 und/oder R7 eine andere Bedeutung als Wasserstoff haben, 1,4-Dihydropyridinderivate der Formel II

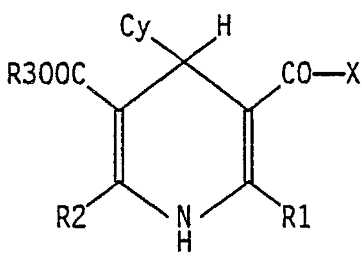

(II),

mit Ephedrinderivaten der Formel III

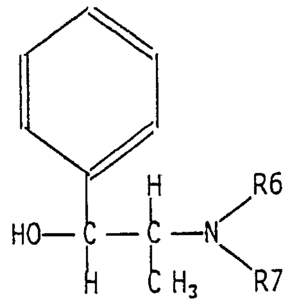

(III),

umsetzt, oder daß man

b) zur Herstellung von Verbindungen I, in denen R6 und/oder R7 eine andere Bedeutung als Wasserstoff haben, Aminocrotonsäurederivate der Formel IV

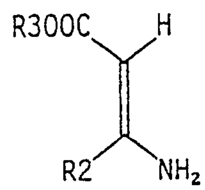

(IV)

mit Acrylsäurederivaten der Formel V

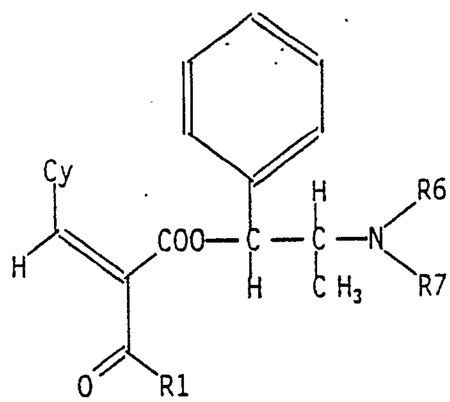

(V)

umsetzt, oder daß man

c) zur Herstellung von Verbindungen I, in denen R6 Wasserstoff bedeutet, aus Verbindungen der Formel I, in denen R6 eine in der Peptidchemie gebräuchliche N-Schutzgruppe darstellt, die Schutzgruppe abspaltet, oder daß man

d) zur Herstellung von Verbindungen I, in denen R6 und R7 eine andere Bedeutung als Wasserstoff haben, Verbindungen der Formel VI

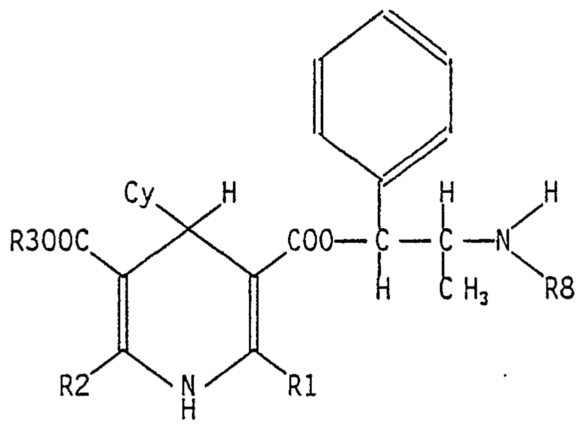

(VI)

worin R8 die Bedeutung von R6 oder R7 (mit Ausnahme der Bedeutung Wasserstoff) hat, mit Verbindungen der Formel VII

Z-R9 (VII)

worin R9 die Bedeutung von R7 oder R6 (mit Ausnahme der Bedeutung Wasserstoff) hat und Z eine Fluchtgruppe darstellt, umsetzt und gewünschtenfalls anschliessend an die Umsetzungen nach a, b, c oder d erhaltene Salze in die freien Basen oder erhaltene Basen in die Salze überführt, wobei Cy, R1, R2, R3, R4, R5, R6 und R7 (sofern nicht anders angegeben) die in Anspruch 1 angegebenen Bedeutungen haben und X und Z Fluchtgruppen darstellen.

9. Arzneimittel enthaltend eine oder mehrere Verbindungen nach einem oder mehreren der Ansprüche 1 bis 7 und/oder ihre pharmakologisch verträglichen Salze.

10. Verbindungen nach einem oder mehreren der Ansprüche 1 bis 7 und ihre pharmakologisch verträglichen Salze zur Anwendung bei der Behandlung und/oder Prophylaxe von Hypertonie, koronaren Herzkrankheiten, peripheren und cerebralen Zirkulationsstörungen und/oder Krankheiten, die auf einer erhöhten Wasser- oder Natriumretention beruhen.

Europäisches Patentamt

# EUROPÄISCHER RECHERCHENBERICHT

Nummer der Anmeldung

EP 89 10 4076

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP-A-0 179 386 (BAYER AG)<br>* Seite 45, Beispiel 53, Seite 72, Ansprüche 9,10 *<br>--- | 1,9 | C 07 D 211/90<br>A 61 K 31/445 |
| D,X | EP-A-0 222 702 (CIBA-GEIGY AG)<br>* Seite 20, Zeilen 22-24; Seite 5, Zeilen 35-46 *<br>--- | 1,9 | |
| D,Y | EP-A-0 088 903 (YOSHITOMI PHARMACEUTICAL IND.)<br>* Seite 31, Anspruch 1, Seite 32, Ansprüche 2,5,6, Seite 3, Zeilen 8-11 *<br>--- | 1,9 | |
| D,X | EP-A-0 060 897 (YOSHITOMI PHARMACEUTICAL)<br>* Seite 12, Zeilen 11-15, Seite 16, Beispiele 7-11, Seite 8, Zeilen 17-20, Seite 20, Anspruch 9 *<br>--- | 1,9 | |
| D,Y | EP-A-0 244 595 (BYK GULDEY LOMBERG)<br>* Seite 47, Anspruch 1, Seite 44, Zeilen 9-17 *<br>--- | 1,9 | **RECHERCHIERTE SACHGEBIETE (Int. Cl.4)** |
| D,Y | EP-A-0 128 010 (TEIJIN LTD)<br>* Seite 54, Beispiele 8,9; Seite 5, Zeilen 1-6,26-29 *<br>--- | 1,9 | C 07 D 211/00<br>A 61 K 31/00 |
| P,A | EP-A-0 280 239 (SANWA KAGAKU CO)<br>* Seite 11, Anspruch 1, Seite 13, Anspruch 13 *<br>----- | 1,9 | |

Der vorliegende Recherchenbericht wurde für alle Patentansprüche erstellt

| Recherchenort | Abschlußdatum der Recherche | Prüfer |
|---|---|---|
| BERLIN | 06-06-1989 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)